(19) Europäisches Patentamt
European Patent Office
Office européen des brevets

(11) Veröffentlichungsnummer: **0 098 975**
**B1**

(12) # EUROPÄISCHE PATENTSCHRIFT

(45) Veröffentlichungstag der Patentschrift: **18.09.85**

(21) Anmeldenummer: **83105674.2**

(22) Anmeldetag: **09.06.83**

(51) Int. Cl.[4]: **A 61 K 35/64** // (A61K35/64, 31:66, 31:55, 31:195)

(54) **Biostimulierendes Mittel.**

(30) Priorität: **09.06.82 DE 3221827**

(43) Veröffentlichungstag der Anmeldung: **25.01.84 Patentblatt 84/04**

(45) Bekanntmachung des Hinweises auf die Patenterteilung: **18.09.85 Patentblatt 85/38**

(84) Benannte Vertragsstaaten: **AT BE FR GB NL SE**

(56) Entgegenhaltungen:
**UNLISTED DRUGS, Band 11, 1959, Chatham, New Jersey, USA**
**DICTIONNAIRE VIDAL, 50. Ausgabe, 1974, O.V.P., Paris, FR.**
**ROTE LISTE, 1961, Editio Cantor, Aulendorf/Württ., DE.**

(73) Patentinhaber: **DSO "PHARMACHIM"**
**Iliensko Chaussée 8**
**Sofia (BG)**

(72) Erfinder: **Kirov, Michail Stefanov, Dr. med.**
**Pelo Pelovski-Str Sona b 5, Bl 5, Vchod A, App 15**
**Sofia (BG)**
Erfinder: **Burov, Pentscho Varbanov, Dipl.-Ing.**
**Komplex Hadji Dimiter, Block 108-A**
**Sofia (BG)**
Erfinder: **Kovatschev, Ivan Christov, Dipl.-Ing.**
**Komplex Serdika, Block 6-2**
**Sofia (BG)**
Erfinder: **Radoslavov, Ivan Christov, Dipl.-Ing.**
**Komplex Borovo, Block 230**
**Sofia (BG)**

(74) Vertreter: **von Füner, Alexander, Dr. et al**
**Patentanwälte v. Füner, Ebbinghaus, Finck Mariahilfplatz 2 & 3**
**D-8000 München 90 (DE)**

EP 0 098 975 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein biostimulierendes Mittel, das für die Genesungszeit (Rekonvaleszenz) des menschlichen Organismus nach schweren und langen Erkrankungen bestimmt ist, sowie auch für Sportler, Soldaten und alle, deren Tätigkeit mit großen physischen Belastungen und intensivem Verbrauch an Muskel- und Nervenenergie verbunden ist.

Biostimulierende Mittel-Präparate, die Vitamine einzeln wie auch in Kombination enthalten, sind bekannt. Am häufigsten werden die Vitamine A, B, C, D, E und andere in Kombination benutzt (Chemie der Vitamine-Beresovskij, N.A., Moskau, 1976). Die Nachteile dieser Präparate bestehen vorwiegend in ihrer geringen Wirkung und in den Nebenerscheinungen, die bei Überdosierung der normalen Dosen auftreten.

Es sind Biostimulantia auf phytochemischer Basis, wie zum Beispiel Extrakt von Holunder u. a. bekannt (Urheberschein Nr. 340 422, 9.VI.69, UdSSR). Deren Nachteil besteht darin, daß sie keine direkte Wirkung auf die Muskelarbeit, sondern nur auf das zentrale und periphere Nervensystem ausüben, weshalb ihr Effekt wesentlich geringer ist.

Es sind weitere Biostimulantia microbialer Herkunft bekannt, die eine direkte, aber unwesentliche Wirkung auf die Muskelarbeit besitzen. Deren Nachteil besteht wiederum darin, daß sie keine entsprechende Wirkung auf das zentrale und periphere Nervensystem ausüben (CH-A-600 884).

Eiweißhydrolysate als Biostimulantia, z. B. Blut-, Kasein- und andere Hydrolysate, sind ebenso bekannt (JP-B-596 839). Deren Nachteil ist, daß der entsprechende Effekt sehr langsam auftritt, weil man auf den Einschluß der freien Aminosäuren in den Stoffaustausch und anschliessend auf eine entsprechende Wirkung auf die Muskulatur hofft. Dieser Einschlußmechanismus, insbesondere, was die Hydrolysate betrifft, wird immer noch diskutiert.

Es ist ein weiterer Biostimulator bekannt, der Kaseinhydrolysat, Succinat, Fumarat, Kreatin, Natrium-Glyzerophosphat und Vitamin C enthält. Er stellt eine Kombination aus Eiweißhydrolysat, Vitamin, zwei Stoffen (Succinat, Fumarat), die den Krebs-Cyclus bewirken, und Kreatin dar (Dopings, Biostimulantia, Sport, J.M. Afer, „Medizin und Sport", 1979). Ein Hauptnachteil dieses Biostimulators ist, daß in der Rezeptur der entsprechende Stoff fehlt, welcher eine adäquante Funktion des Nervensystems mit der Muskelfunktion sichern kann. Die Resultate einer peroralen Einführung von Succinat und Fumarat in den Krebscyclus vorauszusehen, ist schwierig und noch schwieriger die Wirkung des gesamten Präparats, weil ein der Zeit nach unterschiedlicher Wirkungseinsatz der einzelnen Komponente nicht zu dem gewünschten Resultat führen kann.

Die Aufgabe der Erfindung besteht darin, ein biostimulierendes Mittel zu erarbeiten, welches dem peripheren und zentralen Nervensystem die Möglichkeit gibt, bei einer entsprechenden Belastung und Überbelastung des Muskelsystems des Menschen mit einer adäquaten Aktivität zu reagieren.

Die Aufgabe der Erfindung wird durch ein biostimulierendes Mittel gelöst, enthaltend Kreatin und Eiweißhydrolysat, das dadurch gekennzeichnet ist, daß als Eiweißhydrolysat lyophylisiertes Hydrolysat von Weiselfuttersaft enthalten ist, das Mittel noch ein Calcium-Magnesiumsalz der Inositphosphorsäure und Galantamin-Hydrobromid mit folgendem Komponentenverhältnis enthält: Kreatin — von 40 bis 45 Gew. %, Calcium-Magnesiumsalz der Inositphosphorsäure — von 42 bis 46 Gew. %, lyophylisiertes Hydrolysat von Weiselfuttersaft — von 10 bis 12 Gew. %, und Galantamin Hydrobromid — von 1 bis 3 Gew. %.

Die Vorteile des erfindungsgemäßen biostimulierenden Mittels bestehen darin, daß das Mittel direkt auf die Energiebilanz des menschlichen Organismus wirkt, indem es eine entsprechende stimulierende Wirkung auf das zentrale und periphere Nervensystem — bei Kranken während der Rekonvaleszenzperiode, bei Sportlern mit großer Muskelbelastung, bei Soldaten, während Übungen und Training, ausübt.

Beispiel 1

Ein homogenisiertes Gemisch aus Kreatin, lyophylisiertem Weiselfuttersaft-(Gelée royale)-hydrolysat, Calcium-Magnesiumsalz der Inositphosphorsäure und Galantamin-Hydrobromid wird mittels einer automatischen Kapselmaschine in Kapseln von 220 mg dosiert. Der beste Effekt wird bei dem nachstehend angeführten Komponentenverhältnis erreicht: Kreatin — 90 mg, lyophylisiertes Hydrolysat von Weiselfuttersaft — 20 mg, Calcium-Magnesiumsalz der Inositphosphorsäure — 92 mg und Galantamin-Hydrobromid — 5 mg. Die Kapseln werden peroral eingenommen. Diese Applikationsform ist vorwiegend für die Rekonvaleszenzperiode des Organismus nach Erkrankungen vorgesehen, wodurch die Dosierung und die Dauer der Behandlung vom Zustand des Kranken bestimmt werden.

Beispiel 2

Mittels eines automatischen Dosierapparats für Sirupe (Dispensor), werden 10 ml-Flakons mit 6 ml Sirup folgender Zusammensetzung gefüllt: Sirupus Simplex — 6 ml; Nipagin — 0,2 %; Sambuctus Nigra — 0,1 %, und naturales Fruchtaroma (Erdbeere, Himbeere) — 0,1 %.

Die Flakons werden mittels eines Automaten mit sterilen konkaven Kunststoffstöpseln von 300 mg verschlossen. Mit Hilfe eines Dosierapparats für pulverartige Substanzen „Fratelli Canazi" wird jeder Stöpsel mit 90 mg Kreatin, 92 mg Calcium-Magnesiumsalz der Inositphosphorsäure, 20 mg lyophylisiertes Hydrolysat von Weiselfuttersaft (Gelée royale) und 5 mg Galantamin-Hydrobromid als homogenisiertes Gemisch gefüllt. Auf einem beweglichen Förderband sind Stöpselbohrer angeordnet, durch welche die Substanz hermetisch verschlossen wird. Dieses Mittel wird peroral eingenommen, indem man den Stöpselbohrer mit dem Finger drückt, wonach die Substanz mit dem Sirup durch leichtes Schütteln vermischt wird.

Das Einschließen der einzelnen Komponenten in die Energiebilanz des Organismus erfolgt nach folgendem Schema

$$ATF + C \overset{CFC}{\rightleftarrows} ADF + CF$$

Der Biostimulator als ein Komplex von vier sinnvoll dosierten Komponenten zeigte bei den durchgeführten klinischen und paraklinischen Prüfungen bei dynamischen Verhältnissen eine sehr starke Erhöhung der Aktivität der quergestreiften Muskulator, des Herzmuskels und des zentralen und peripheren Nervensystems. Es zeigt eine 1/20 $LD_{50}$ für Ratten ($LD_{50}$ = 4 100 mg/kg).

Sportler und Soldaten müssen das Präparat mindestens 1 Stunde vor der physischen Belastung einnehmen und Kranke während der Rekonvaleszenzperiode — nach ärztlicher Vorschrift für den gegebenen Fall.

**Patentanspruch**

Biostimulierendes Mittel, enthaltend Kreatin und Eiweißhydrolysat, dadurch gekennzeichnet, daß als Eiweißhydrolysat lyophylisiertes Hydrolysat von Weiselfuttersaft enthalten ist, das Mittel noch ein Calcium-Magnesiumsalz der Inositphosphorsäure und Galantamin-Hydrobromid mit folgendem Komponentenverhältnis enthält :

Kreatin : von 40 bis 45 Gew. %,
Calcium-Magnesiumsalz der Inositphosphorsäure : von 42 bis 46 Gew. %,
lyophylisiertes Hydrolysat,
von Weiselfuttersaft : von 10 bis 12 Gew. %, und
Galantamin Hydrobromid : von 1 bis 3 Gew. %.

**Claim**

A biostimulant comprising creatine and the hydrolyzate of protein, characterized in that as the hydrolyzate of protein there is contained lyophylized hydrolyzate of Royal Jelly and that the biostimulant furthermore contains calcium-magnesium salt of inositephosphoric acid and galanthamine-hydrobromide, whereby the ratio of the individual components is the following :

40 to 45 % by weight of creatine ;

42 to 46 % by weight of calcium-magnesium salt of inositephosphoric acid ;

10 to 12 % by weight of lyophylized hydrolyzate of Royal Jelly ;

1 to 3 % by weight of galanthamine-hydrobromide.

**Revendication**

Agent biostimulant contenant de la créatine et de l'albumine hydrolysée, caractérisée en ce que de la gelée royale hydrolysée lyophilisée est contenue comme albumine hydrolysée et que l'agent contient en outre un sel de calcium et magnésium de l'acide phosphorique d'inosite et de l'hydrobromure de galantamine, le rapport des components particuliers étant :

de 40 à 45 % en poids de créatine,

de 42 à 46 % de sel de calcium et magnésium de l'acide phosphorique d'inosite,

de 10 à 12 % en poids de gelée royale hydrolysée et

de 1 à 3 % en poids d'hydrobromure de galantamine.